(19) Europäisches Patentamt
European Patent Office
Office européen des brevets

(11) Veröffentlichungsnummer: **0 200 677 B1**

(12) **EUROPÄISCHE PATENTSCHRIFT**

(45) Veröffentlichungstag der Patentschrift: **03.04.91**

(51) Int. Cl.⁵: **C07D 213/643**, C07F 7/08, C07F 9/40, A01N 43/40

(21) Anmeldenummer: **86810137.9**

(22) Anmeldetag: **24.03.86**

Die Akte enthält technische Angaben, die nach dem Eingang der Anmeldung eingereicht wurden und die nicht in dieser Patentschrift enthalten sind.

(54) **3-Fluorpyridyl-2-oxy-phenoxy-Derivate mit herbizider Wirkung.**

(30) Priorität: **01.04.85 CH 1401/85**

(43) Veröffentlichungstag der Anmeldung:
**10.12.86 Patentblatt 86/45**

(45) Bekanntmachung des Hinweises auf die Patenterteilung:
**03.04.91 Patentblatt 91/14**

(84) Benannte Vertragsstaaten:
**BE CH DE FR GB IT LI NL**

(56) Entgegenhaltungen:
**EP-A- 0 000 483**
**EP-A- 0 006 608**
**EP-A- 0 021 453**
**EP-A- 0 040 478**
**EP-A- 0 083 556**
**EP-A- 0 096 354**
**EP-A- 0 097 460**
**EP-A- 0 104 715**
**EP-A- 0 112 531**
**EP-A- 0 113 831**
**EP-A- 0 115 823**
**EP-A- 0 128 658**
**EP-A- 0 191 736**
**DE-A- 2 546 251**
**DE-A- 2 909 816**
**DE-A- 3 131 363**
**US-A- 4 670 040**

(73) Patentinhaber: **CIBA-GEIGY AG**
**Klybeckstrasse 141**
**CH-4002 Basel(CH)**

(72) Erfinder: **Schurter, Rolf, Dr.**
**Holzmattstrasse 45**
**CH-4102 Binningen(CH)**
Erfinder: **Diel, Peter J., Dr.**
**Kornfeldstrasse 73**
**CH-4125 Riehen(CH)**

**EP 0 200 677 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der offengelegten Europäischen Patentanmeldung EP-A-83 556 sind bereits 3-Fluoropyridyl-2-oxy-phenoxy-Derivate mit herbizider Wirkung beschrieben,welche der Formel

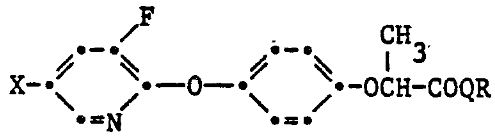

entsprechen, worin Q Sauerstoff oder Schwefel, R unter anderem Wasserstoff, ein Metall- oder Ammoniumion der einen gegebenenfalls substituierten $C_1$-$C_9$-Alkyl, $C_3$-$C_9$-Cycloalkyl, $C_3$-$C_9$-Alkenyl- oder $C_3$-$C_9$-Alkinylrest, einen $C_3$-$C_9$-Cycloalkenylrest oder einen Iminorest und X Chlor, Brom oder Jod bedeuten.

Diese Verbindungen zeigten gute selektiv-herbizide Wirkung, vor allem gegen grasartige Unkräuter in Kulturen von Nutzpflanzen, wie Getreide, Mais, Reis, Baumwolle, Soja und Zuckerrüben.

Es hat sich gezeigt, dass eine weitere Gruppe neuer 3-Fluoro-pyridyl-2-oxy-phenoxy-Derivate ausgezeichnete selektiv-herbizide Wirkung im Vor- und Nachauflaufverfahren gegenüber vornehmlich grasartige Unkräutern in Kulturen von Nutzpflanzen besitzen.

Die neuen 3-Fluorpyridyl-2-oxy-phenoxy-Derivate entsprechen der Formel I

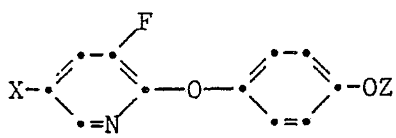 (I),

worin

X ein Halogenatom oder die Trifluormethylgruppe und
Z einen Alkancarbonsäurerest $-CH(CH_3)Z^1$, $CH_2Z^2$, oder $-CH(CH_2OCH_3)Z^2$
bedeutet, wobei
$Z^1$ einen Rest $-COQ-G-Y^2$
$Z^2$ dasselbe wie $Z^1$
$Y^2$ einen Rest
- $PO(OR^{14})(O)_pR^{15}$ oder
- $Si-A^7-(O_pR^{12})R^{13}$,
Q, Sauerstoff oder Schwefel
$R^{12}$, $R^{13}$ je $C_1$-$C_6$-Alkyl,
$R^{14}$, $R^{15}$ unabhängig voneinander je Wasserstoff, $C_1$-$C_4$-Alkyl, $C_3$-$C_4$-Alkenyl, $C_3$-$C_4$-Alkinyl, $C_2$-$C_4$-Halogenalkyl, $C_1$-$C_4$-Alkoxy-($C_2$-$C_4$)-alkyl, $C_1$-$C_4$-Cyanoalkyl oder einen unsubstituierten oder durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyano ein-bis zweifach substituierten Phenyl-, Phenyl-($C_1$-$C_4$)-alkyl oder Naphthylrest;
wobei p 0 oder 1 und
$R^{15}$ nicht H bedeutet, wenn
p = 0 ist,
$A^7$ $C_1$-$C_6$-Alkyl, $C_1$-$C_6$-Alkoxy,
G eine $C_1$-$C_3$-Alkylenkette, die 1- bis 2-fach durch einen Substituenten aus der Gruppe Methyl und Phenyl substituiert sein kann
bedeuten.

Die obige Definition von G umfasst Methylen, 1,2-Aethylen und 1,3-Propylen und die durch Substitution von 1 bis 2 Wasserstoff-atomen durch Methyl oder Phenyl davon abgeleiteten Gruppen, wie z.B. Aethyliden, Isopropyliden (2,2-Propyliden), Benzyliden, 1-Phenyl-äthyliden, Diphenylmethylen, 1,2-Propylen, 2,3-Butylen, 1,1-Dimethyl-1,2-äthylen, 1,1-Diphenyl-1,2-äthylen, 1,2-Diphenyl-1,2-äthylen, 1-Methyl-1-phenyl-1,2-äthylen, 1,2-Butylen, 1,3-Butylen, 2,2-Dimethyl-1,3-propylen, 1-Methyl-1-phenyl-1,3-propylen, 1,2-Diphenyl-1,3-propylen und 1,3-Diphenyl-1,3-propylen.

Falls der Alkancarbonsäurerest Z ein asymmetrisch substituiertes Kohlenstoffatom enthält, (wie z.B. $-CH(CH_3)Z^1$) können zwei enantiomere Formen vorliegen. Die Erfindung betrifft sowohl die Racemate wie auch die R- und S-Enantiomeren.

Die erfindungsgemässen 3-Fluorpyridyl-2-oxy-phenoxyderivate zeichnen sich durch eine gute Wirkung gegen mono- und einige dikotyle Unkräuter aus, vor allem wirken sie im Nachauflaufverfahren gegen widrige in Kulturen wie Getreide, Mais, Reis, Soja und Zuckerrüben vorkommende Unkräuter und Ungräser. Besonders wertvoll ist die Möglichkeit mit ihnen Ungräser zu bekämpfen, denen sonst nur schwer beizukommen ist, wie z.B. Avena fatua, Avena sterilis, Alopecurus myosuroides, Lolium perenne, Phalaris sp., Bromus tectorum, verschiedene Setaria- und Panicum Arten. Die Wirkung tritt unter Feldbedingungen bereits bei niederen Aufwandmengen von weniger als 1 kg pro Hektar auf, bei denen die Kulturen nicht oder nur ganz unbedeutend geschädigt werden.

Halogenpyridyloxy-α-phenoxy-propionsäurederivate sind in zahlreichen Publikationen beschrieben worden, siehe beispielsweise die DE-OS 2 546 251, 2 649 706, 2 714 622, 2 715 284, 2 909 816 und 3 131 363 sowie die offengelegten Europäischen Patentanmeldungen EP-A 000 483, 001 473, 021 453, 040 478, 083 556, 112 531, 113 831, 115 823 und 191 736. Darin werden die erfindungsgemässen 3-Fluorpyridyl-2-oxy-phenoxyderivate zum Teil auch in Erwägung gezogen und vom Umfang miterfasst. Solche Verbindungen sind jedoch nie hergestellt oder geprüft worden. Sie unterscheiden sich von den bekannten Halogenpyridyloxy-α-phenoxypropionsäuren durch stärkere herbizide Wirkung und damit der Möglichkeit sie in geringeren Aufwandmengen einzusetzen.

Die EP-A 97 460 beschreibt von den Verbindungen vorliegender Erfindung verschiedene 3-Fluorpyridin-2-yloxy-phenoxy-carbonsäurederivate.

In der EP-A 96 354 sind 2-(Pyridin-2-yloxy-phenoxy)-essig- und -propionsäureester beschrieben, die im Esterteil Phosphor oder Silicium, und im USP 4670 040 solche, die im Esterteil Phosphor enthalten. Diesen gegenüber unterscheiden sich die erfindungsgemässen Verbindungen durch das Fluoratom in 3-Stellung des Pyridinringes.

Bei genügend grosser Aufwandmenge ist auch totalherbizide Wirkung vorhanden. Die Anwendung kann sowohl im Vorauflauf- wie im Hachauflaufverfahren vorgenommen werden. Dabei können die Aufwandmengen in weiten Grenzen schwanken, z.B. zwischen 0,05 bis 5 kg Wirkstoff pro Hektare.

Ferner besitzen die Verbindungen der Formel I günstige wachstumsregulierende Effekte (Wuchshemmung). Insbesondere hemmen sie das Wachstum von Gräsern.

Als sehr aktiv haben sich diejenigen 3-Fluorpyridyl-2-oxy-phenoxy-derivate der Formel I erwiesen, welche der Formel Ib entsprechen

Ferner die 3-Fluorpyridyl-2-oxy-phenoxy Derivate der Formel Ib

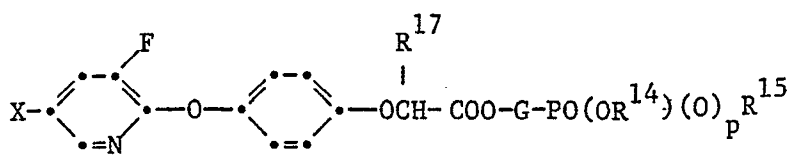 (Ib)

worin X, G, $R^{14}$, $R^{15}$ und p die unter Formel I gegebene Bedeutung haben und $R^{17}$ Wasserstoff, Methyl oder Methoxymethyl bedeutet, besonders diejenigen Verbindungen in deren X Chlor oder Trifluoromethyl, G $C_1$-$C_3$-Alkylen, $R^{14}$ und $R^{15}$ je Methyl oder Aethyl und $R^{17}$ Methyl bedeuten.

Beispiele dafür sind der (2-[4-(5-Chlor-3-fluor-pyridinyl-2-oxy)phenoxy]-propionyl-oxy)-methylphosphonsäure-diäthylester und der (2-[4-(5-Chlor-3-fluor-pyridinyl-2-oxy)phenoxy]-propionyl-oxy)-methylphosphonsäure-dimethylester.

Ebenfalls als aktiv zeigten sich die Verbindungen der Formel If

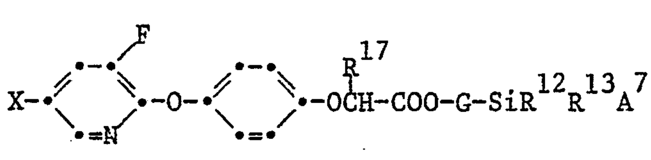 (If)

worin $A^7$, X, G, $R^{12}$, $R^{13}$ und $R^{17}$ die oben gegebenen Bedeutungen haben, ganz besonders diejenigen Verbindungen in denen X Chlor oder Trifluormethyl, G $C_1$-$C_3$-Alkylen und $R^{12}$, $R^{13}$ und $R^{17}$ je Methyl bedeuten.

Beispiel dafür sind der

α- [4-(5-Chlor-3-fluorpyridyl-2-oxy)-phenoxy]-propionsäure-trimethyl-silyl-methylester, der
α-[4-(5-Chlor-3-fluorpyridyl-2-oxy)-phenoxy]-propionsäure-2'-trimethyl-silyl-eth-1'-yl-ester, der
α-[4-(3-Fluor-5-trifluormethyl-pridyl-2-oxy)-phenoxy]-propionsäure-2'-trimethylsilyl-eth-1'-yl-ester und der
α-[4-(3-Fluor-5-trifluormethyl-pyridyl-2-oxy)-phenoxy]-propionsäure-trimethylsilyl-methylester.

Die Herstellung der neuen Verbindungen der Formel I erfolgt nach an sich bekannten Methoden.
Nach einem ersten dieser Verfahren setzt man ein 2,3-Difluorpyridin der Formel II

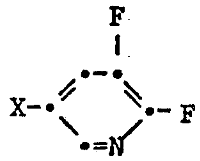

(II),

worin X die unter Formel I gegebene Bedeutung hat, in einem inerten Lösungs- oder Verdünnungsmittel, in Gegenwart der äquimolaren Menge einer Base mit einem 4-Hydroxyphenoxy-α-alkansäurederivat der Formel III um,

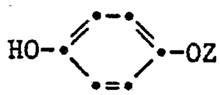

(III),

worin Z die unter Formel I gegebene Bedeutung hat.
Ein anderes Verfahren besteht darin, dass man ein 4-(3-Fluor-pyridyl-2-oxy)-phenol der Formel IV

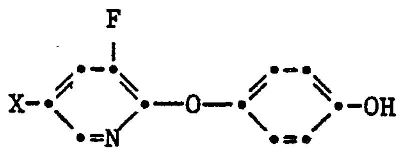

(IV),

worin X die unter Formel I gegebene Bedeutung hat, in einem inerten Lösungs- oder Verdünnungsmittel in Gegenwart der äquimolaren Menge einer Base mit einem Halogenid der Formel V umsetzt

$$Hal - Z \quad (V),$$

worin Hal Chlor oder Brom bedeutet und Z die unter Formel I gegebene Bedeutung hat.
Ein weiteres Verfahren besteht darin, dass man ein 3-Aminopyridyl-2-oxyphenoxy-Derivat der Formel VI

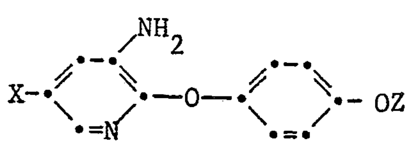

(VI),

worin X und Z die unter Formel I gegebene Bedeutung haben, nach bekannten Methoden in ein Diazoniumsalz und dieses weiter in die Fluorverbindung überführt.
Schliesslich besteht ein Verfahren zur Herstellung der Verbindungen der Formel I darin, dass man ein Säurehalogenid der Formel VII

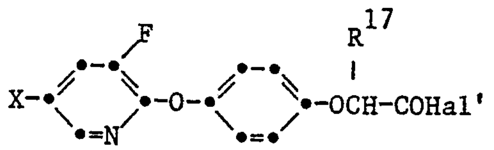

(VII),

worin Hal' Fluor, Chlor oder Brom bedeuten und X und $R^{17}$ die oben angegebene Bedeutung haben, in einem inerten Lösungsmittel oder Verdünnungsmittel in Gegenwart eine äquimolaren Menge einer Base mit einem Alkohol oder Thiol umsetzt.

Unter Alkohol verstehen wir u.a. die Verbindungsklassen HQR und HQ-G-PO($OR^{14}$)(O)$_p$($R^{15}$).

Ein Teil dieser Umsetzungen werden vorteilhafterweise in einem den Reaktionspartnern gegenüber inerten, organischen Lösungs- oder Verdünnungsmittel durchgeführt, wie z.B. einem Alkohol, Ester, Aether, Keton, Dimethylformamid, Dimethylsulfoxyd, Acetonitril, einem Aromaten wie Benzyl, Toluol etc..

Die Reaktionstemperaturen liegen zwischen -10° und +150°C, praktisch aber zwischen der Raumtemperatur und dem Siedepunkt des Lösungsmittels. Die Reaktionsdauer beträgt je nach dem gewählten Ausgangsstoff, dem Lösungsmittel und der Temperatur 1 Stunde bis ca. einen Tag.

Wo ein Halogenatom bei der Reaktion abgespalten wird, sollte die äquimolare Menge eines säurebindenden Mittels eingesetzt werden. Als solches eignet sich im Prinzip jede anorganische oder organische Base, wie z.B. NaOH, KOH, $NaHCO_3$, $K_2CO_3$, K-tert.Butylat, und Amine, wie Trimethylamin, Triäthylamin, Pyridin, 4-Dimethylaminopyridin etc..

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxid etc. löslich sind. Sie sind nicht explosiv oder ätzend und ihre Handhabung bedarf keiner besonderer Vorsichtsmassnahmen.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl äther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxidertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigen-schaften in Betracht. Unter Tensiden sind auch Tensidgemisch zu verstehen.

Geeignete anionische Tenside können sowohl sog. wasserlösliche Seifen wie wasserlösliche synthetische oberflächenaktive Verbindungen sein. Als Seifen sind z.B. die Alkali-, Erdalkali- oder gegebenenfalls substituierten Ammoniumsalze von höheren Fettsäuren ($C_{10}$-$C_{20}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurin-salze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alyklarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehyd-kondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie · Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
H. Stache "Tensid Taschenbuch" 2. Aufl., C. Hanser Verlag, München Wien 1981.
M. and J. Ash, "Encyclopedia of Surfactants" Vol I-III Chemical Publishing Co. New York, 1980-81.

Diese Zubereitungen enthalten in der Regal 0,1 bis 99%, insbesondere 0,1 bis 95%, Nirkstoff der Formel I, 1 bis 99% eines festen oder flüssigen Zusatzstoffes und 0 bis 25%, insbesondere 0,1 bis 25% eines Tensides.

Während als Handelsware eher Konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die folgende Beispiel beschreibt im Detail die Herstellung der erfindungsgemässen 3-Fluorpyridyl-2-oxy-phenoxyderivate der Formel I. Weitere in analoger Weise erhaltene erfindungsgemässe Ester sind in den sich dem Beispiel anschliessenden Tabellen aufgeführt. Die Temperaturen sind in Grad Celsius angegeben, während sich Prozentangaben auf das Gewicht beziehen.

Beispiel 1: Herstellung von 2-[4-(3-Fluor-5-trifluormethylpyridyl-2-oxy)-phenoxy]-propionsäure[2-(0,0-dimethylphosphonyl)äthyl]ester

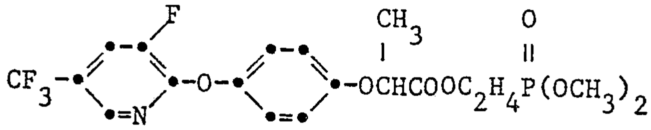

7,7 g (0,05 mol) 2-Hydroxyäthyl-0,0-dimethylphosphonat werden zusammen mit 6,9 ml (0,05 mol) Triäthylamin und einer Spatelspitze 4-Piperidinopyridin in 130 ml Aethylacetat vorgelegt. Unter Rühren tropft man bei Raumtemperatur 17,9 g (0,05 mol) 2-[4-(3-Fluor-5-trifluormethylpyridyl-2-oxy)-phenoxy]-propionsäurechlorid in 20 ml Aethylacetat zu. Es wird 1 Stunde bei 30-35 °C und 1 Stunde bei Rückfluss gerührt, dann lässt man abkalten, filtriert, behandelt das Filtrat mit Aktivkohle, trocknet über Natriumsulfat und dampft ein. Man erhält 22,1 g braunes Oel, das nun mit $CH_2Cl_2/CH_3OH$ 95:5 über Kieselgel chromatographiert wird. Ausbeute: 17,5 g hellgelbes Oel, $n_D^{30}$ 1.4973

In analoger Weise zu diesen Beispielen werden folgende Verbindungen hergestellt:

Tabelle **1**:

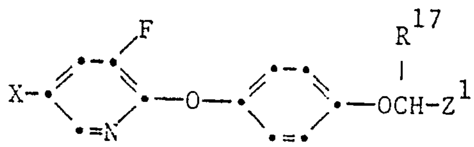

| Nr. | X | $R^{17}$ | $Z^1$ | physikalische Daten |
|---|---|---|---|---|
| **1**.01 | Cl | $CH_3$ | $-COOCH_2P(O)(OC_2H_5)_2$ | |
| **1**.02 | Cl | $CH_3$ | $-COOCH_2P(O)(OCH_3)_2$ | $n_D^{30}$ 1,5317 |
| **1**.03 | Cl | $CH_3$ | $-COOCH_2CH_2P(O)(OC_2H_5)_2$ | |
| **1**.04 | Cl | $CH_3$ | $-COOCH_2CH_2P(O)(OCH_3)_2$ | |
| **1**.05 | Cl | $CH_3$ | $-COOCH_2CH_2CH_2P(O)(OC_2H_5)_2$ | |
| **1**.06 | Cl | $CH_3$ | $-COOCH_2-CH(CH_3)P(O)(OC_5H_2)_2$ | |
| **1**.07 | Cl | $CH_3$ | $-COOCH_2P(O)(OCH_3)_2$ | |
| **1**.08 | Cl | H | $-COOCH_2P(O)(OCH_3)_2$ | |
| **1**.09 | Cl | $CH_3$ | $-COOCH(CH_3)P(O)(OCH_3)_2$ | |
| **1**.10 | Cl | $CH_3$ | $-COOC(CH_3)_2P(O)(OCH_3)_2$ | |
| **1**.11 | Cl | $CH_3$ | $-COOCH_2P(O)(OCH_3)CH_3$ | |
| **1**.12 | $CF_3$ | $CH_3$ | $-COOCH_2P(O)(OC_2H_5)CH_3$ | $n_D^{30}$ 1,4992 |
| **1**.13 | Cl | $CH_3$ | $-COOCH(CH_3)P(O)(OCH_3)CH_3$ | |
| **1**.14 | Cl | $CH_3$ | $-COOCH(CH_3)P(O)(OC_2H_5)_2$ | $n_n^{30}$ 1,5140 |
| **1**.15 | $CF_3$ | $CH_3$ | $-COOCH_2P(O)(OCH_3)_2$ | |
| **1**.16 | $CF_3$ | $CH_3$ | $-COOCH_2CH_2P(O)(OCH_3)_2$ | $n_D^{30}$ 1,4973 |

Tabelle 2

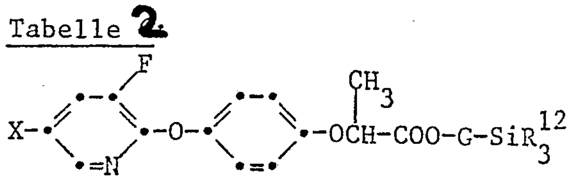

| Nr. | X | G | $R^{12}$ | Bemerkungen | Physikalische Eigenschaften |
|---|---|---|---|---|---|
| 2.01 | Cl | $-CH_2-CH_2-$ | $CH_3$ | 2R-Enantiomeres | $n_D^{35}$ 1,5181 $[\alpha]_D^{20}$ = +37,0° (Aceton) |
| 2.02 | Cl | $-CH_2-$ | $CH_3$ | 2R-Enantiomeres | $n_D^{35}$ 1,5201 $[\alpha]_D^{20}$ = +41,3° (Aceton) |
| 2.03 | $CF_3$ | $-CH_2-$ | $CH_3$ | Racemat | Smp. 61-63°C |
| 2.04 | $CF_3$ | $-CH_2-CH_2-$ | $CH_3$ | Racemat | $n_D^{35}$ 1,4842 |
| 2.05 | Cl | $-CH_2-CH_2-$ | $CH_3$ | Racemat | $n_D^{35}$ 1,5201 |
| 2.06 | Cl | $-CH_2-$ | $CH_3$ | Racemat | $n_D^{35}$ 1,5155 |
| 2.07 | Cl | $-CH_2-$ | $C_2H_5$ | | |
| 2.08 | Cl | $-CH_2-CH_2-$ | $C_2H_5$ | | |
| 2.09 | Cl | $-CH(CH_3)-CH_2-$ | $CH_3$ | | |
| 2.10 | Cl | $-CH_2CH(CH_3)-$ | $CH_3$ | | |
| 2.11 | Cl | $-CH(CH_3)-$ | $CH_3$ | | |
| 2.12 | $CF_3$ | $-CH(CH_3)-$ | $CH_3$ | | |

Beispiel 2:

Herstellung einer Formulierung mit flüssigen Wirkstoffen der Formel I (% = Gewichtsprozent)

| Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| Lösungen | a) | b) | c) | d) |
|---|---|---|---|---|
| Wirkstoff gemäss der Tabellen 1 und 2 | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenze 160-190°C) | - | - | 94% | - |

Die Lösungen sind zur Anwendung in Form kleinster Tropfen geeignet.

| Granulate | a) | b) |
|---|---|---|
| Wirkstoff der Tabellen 1 und 2 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| Stäubemittel | a) | b) |
|---|---|---|
| Wirkstoff der Tabellen 1 und 2 | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| Spritzpulver | a) | b) |
|---|---|---|
| Wirkstoff der Tabellen 1 und 2 | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff der Tabellen 1 und 2 | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Stäubemittel | a) | b) |
|---|---|---|
| Wirkstoff der Tabellen 1 und 2 | 5% | 8% |
| Talkum | 95% | - |
| Kaolin | - | 92% |

Man erhält anwendungsfertige Stäubemittel, indem der Wirkstoff mit den Trägerstoffen vermischt und auf einer geeigneten Mühle vermahlen wird.

| Extruder Granulat | |
|---|---|
| Wirkstoff der Tabellen 1 und 2 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

Umhüllungs-Granulat

| | |
|---|---|
| Wirkstoff der Tabellen 1 und 2 | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Suspensions-Konzentrat

| | |
|---|---|
| Wirkstoff der Tabellen 1 | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%-ige wässrige Formaldehyd-Lösung | 0,2% |
| Silikonöl in Form einer 75%-igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Prüfung der herbiziden Wirkung.

Pre-emergente Herbizid-Wirkung (Keimhemmung)

Im Gewächshaus wird unmittelbar nach der Einsaat der Versuchspflanzen in Töpfe die Erdoberfläche mit einer wässrigen Dispersion des Wirkstoffes erhalten aus einem 25%-igen Emulsionskonzentrat oder aus einem 25%-igen Spritzpulver mit Wirkstoffen, die wegen ungenügender Löslichkeit nicht als Emulsionskonzentrat hergestellt werden können, gespritzt. Es werden verschiedene Konzentrationen angewendet und die Menge Wirkstoff wird in kg pro Hektar ausgerechnet. Die Töpfe werden dann im Gewächshaus bei 22-25 °C und 50-70% relativer Luftfeuchtigkeit gehalten und regelmässig bewässert. Der Versuch wird nach 3 Wochen ausgewertet. Die geprüften Verbindungen der Tabelle 1 zeigen gute bis sehr gute Phytotoxizität gegenüber den geprüften Monokolyledonen.

Post-emergente Herbizid-Wirkung (Kontaktherbizid)

Eine grössere Anzahl Unkräuter und Kulturpflanzen, sowohl monokotyle wie dicotyle, wurden im Gewächshaus in Töpfen gezogen und nach dem Auflaufen (im 4- bis 6-Blattstadium) mit einer wässerigen Wirkstoffdispersion in verschiedenen Dosierungen, ausgedrückt in kg Wirksubstanz pro Hektare, auf die Pflanzen gespritzt und diese bei 24-26 °C und 45-60% relativer Luftfeuchtigkeit gehalten. Der Versuch wird 2 Wochen nach der Behandlung ausgewertet. Auch hier wirken die geprüften Verbindungen der Tabellen 1

und 2 ausgezeichnet gegen die monokotylen Ungräser.

Wuchshemmung bei Gräsern

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit einer wässerigen Spritzbrühe der Verbindungen der Formel I bespritzt. Die Wirkstoffmenge betrug umgerechnet 0,05 - 2 kg Aktivsubstanz pro Hektar. 10 und 21 Tage nach der Applikation wurde das Wachstum der Gräser mit derjenigen einer unbehandelten Kontrolle verglichen. Die geprüften Verbindungen der Tabellen 1 und 2 vermindern bei der Aufwandmenge von 0,05 kg pro Hektare das Wachstum der Gräser um 18 - 32%.

## Ansprüche

**1.** 3-Fluor-pyridyl-2-oxy-phenoxy Derivate der Formel I

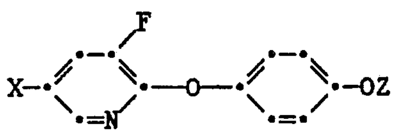

(I)

worin

| | |
|---|---|
| X | ein Halogenatom oder die Trifluormethylgruppe und |
| Z | einen Alkancarbonsäurerest $-CH(CH_3)Z^1$, $CH_2Z^2$ oder $CH(CH_2)OCH_3)Z^2$ bedeutet wobei |
| $Z^1$ | einen Rest $-COQ-G-Y^2$, |
| $Z^2$ | dasselbe wie $Z^1$, |
| $Y^2$ | einen Rest $-PO(OR^{14})OR^{15}$ oder $SiA^7(O)pR^{12})R^{13}$ |
| Q | Sauerstoff oder Schwefel, |
| $R^{12}$ | und $R^{13}$ je $C_1$-$C_6$-Alkyl |
| $R^{14}$, | $R^{15}$ unabhängig voneinander je Wasserstoff, $C_1$-$C_4$-Alkyl, $C_3$-$C_4$-Alkenyl, $C_3$-$C_4$-Alkinyl, $C_2$-$C_4$-Halogenalkyl, $C_1$-$C_4$-Alkoxy-($C_2$-$C_4$)-alkyl, $C_1$-$C_4$-Cyanoalkyl oder einen unsubstituierten oder durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyano einbis zweifach substituierten Phenyl-, Phenyl-($C_1$-$C_4$)-alkyl oder Naphthylrest; wobei p 0 oder 1 ist und $R^{15}$ nicht H bedeutet, wenn p = 0 ist, |
| $A^7$ | $C_1$-$C_6$-Alkyl, $C_1$-$C_6$-Alkoxy, |
| G | eine $C_1$-$C_3$-Alkylenkette, die 1- bis 2-fach durch einen Substituenten aus der Gruppe Methyl, Phenyl substituiert sein kann, bedeuten. |

**2.** 3-Fluor-pyridyl-2-oxy-phenoxy-Derivate gemäss Anspruch 1 der Formel (Ib),

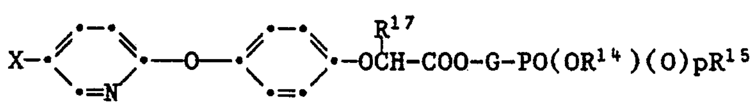

(Ib)

worin X, G, $R^{14}$ $R^{15}$ und p die im Anspruch 1 gegebene Bedeutung haben, während $R^{17}$ Wasserstoff, Methyl oder Methoxymethyl bedeutet.

**3.** 3-Fluor-pyridyl-2-oxy-phenoxy-Derivate gemäss Anspruch 2 der Formel Ib, worin X Chlor oder Trifluormethyl, G $C_1$-$C_3$ Alkylen $R_{14}$ und $R_{15}$ je Methyl oder Ethyl und $R_{17}$ Methyl bedeuten.

4. (2-[4-(5-Chlor-3-fluor-pyridyl-2-oxy)phenoxy]propionyl-oxy)-methylphosphonsäure-diäthylester gemäss Anspruch 1.

5. (2-[4-(5-Chlor-3-fluor-pyridyl-2-oxy)phenoxy]propionyl-oxy)-methylphosphonsäuredimethylester gemäss Anspruch 1.

6. 3-Fluor-pyridyl-2-oxy-phenoxy-Derivate gemäss Anspruch 1 der Formel If,

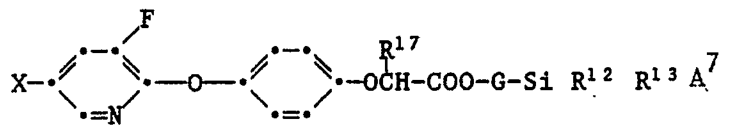 (If)

worin $A^7$ X, G, $R^{12}$ und $R^{13}$ die im Anspruch 1 gegebene Bedeutung haben, während $R^{17}$ Wasserstoff, Methyl oder Methoxymethyl bedeutet.

7. 3-Fluor-pyridyl-2-oxy-phenoxy-Derivate gemäss Anspruch 6 der Formel If, worin X Chlor oder Trifluormethyl, G $C_1$-$C_3$ Alkylen und $R^{12}$, $R^{13}$ und $R^{17}$ je Methyl bedeuten.

8. 2-[4-(5-Chlor-3-fluor-pyridyl-2-oxy)phenoxy]propionsäure-trimethylsilyl-methylester gemäss Anspruch 1.

9. 2-[4-(5-Chlor-3-fluor-pyridyl-2-oxy)phenoxy]propionsäure-2'-trimethylsilyl-eth-1'-yl-ester gemäss Anspruch 1.

10. (2R)-2-[4-(5-Chlor-3-fluor-pyridyl-2-oxy)phenoxy]propionsäure-trimethylsilyl-methylester gemäss Anspruch 1.

11. (2R)-2-[4-(5-Chlor-3-fluor-pyridyl-2-oxy)phenoxy]propionsäure-2'-trimethylsilyl-eth-1'-yl-ester gemäss Anspruch 1.

12. (2R)-2-[4-(3-Fluor-5-trifluormethyl-pyridyl-2-oxy)phenoxy]-propionsäure-2'-trimethylsilyl-eth-1'-yl-ester gemäss Anspruch 1.

13. (2R)-2-[4-(3-Fluor-5-trifluormethyl-pyridyl-2-oxy)phenoxy]-propionsäure-trimethylsilyl-methylester gemäss Anspruch 1.

14. 2-[4-(3-Fluor-5-trifluormethyl-pyridyl-2-oxy)phenoxy]propionsäure-2'-trimethylsilyl-eth-1'-yl-ester gemäss Anspruch 1.

15. 2-[4-(3-Fluor-5-trifluormethyl-pyridyl-2-oxy)phenoxy]propionsäure-trimethylsilyl-methylester gemäss Anspruch 1.

16. Verfahren zur Herstellung der 3-Fluor-pyridyl-2-oxy-phenoxy-Derivate der Formel I, im Anspruch 1, dass man ein 2,3-Difluorpyridin der Formel II

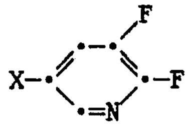 (II)

worin X die im Anspruch 1 gegebene Bedeutung hat, in einem inerten Lösungs- oder Verdünnungsmittel, in Gegenwart der äquimolaren Menge einer Base mit einem 4-Hydroxy-phenoxy-α-alkansäurederivat der Formel III umsetzt,

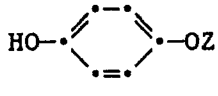 (III)

worin Z die im Anspruch 1 gegebene Bedeutung hat.

**17.** Verfahren zur Herstellung der 3-Fluor-pyridyl-2-oxy-phenoxy Derivate der Formel I, Anspruch 1, dadurch gekennzeichnet, dass man ein 4-(3-Fluor-pyridyl-2-oxy)-phenol der Formel IV

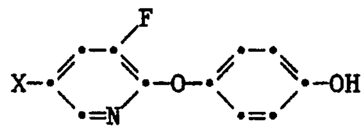

(IV)

worin X die im Anspruch 1 gegebene Bedeutung hat, in einem inerten organischen Lösungs- oder Verdünnungsmittel, in Anwesenheit der äquimolaren Menge einer Base mit einem Halogenid der Formel V umsetzt,

Hal-Z (V)

worin Hal Chlor oder Brom bedeutet und Z die unter Formel I, Anspruch 1, gegebene Bedeutung hat.

**18.** Verfahren zur Herstellung der 3-Fluor-pyridyl-2-oxy-phenoxy-Derivate der Formel I, Anspruch 1, dadurch gekennzeichnet, dass man ein 3-Aminopyridyl-2-oxy-phenoxyderivat der Formel VI

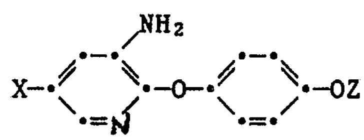

(VI)

worin X und Z die unter Formel I, Anspruch 1 gegebenen Bedeutungen haben, in ein Diazoniumsalz und dieses weiter in die Fluorverbindung überführt wird.

**19.** Verfahren zur Herstellung der 3-Fluor-pyridyl-2-oxy-phenoxy-Derivate der Formel I, Anspruch 1, dadurch gekennzeichnet, dass man ein Säurehalogenid der Formel VII

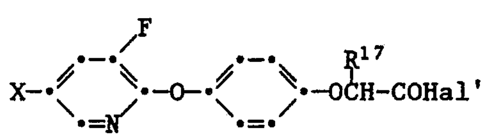

(VII)

worin Hal' Fluor, Chlor oder Brom bedeuten, X die im Anspruch 1 gegebene Bedeutung hat und $R^{17}$ Wasserstoff, $C_1$-$C_2$-Alkyl oder Methoxymethyl bedeutet, in einem inerten Lösungs- oder Verdünnungsmittel, in Gegenwart einer äquimolaren Menge einer Base mit einem Alkohol oder Thiol. der Formel HQ-G-$Y^2$ umsetzt, worin G, Q und $Y^2$ die unter Formel I, Anspruch 1 gegebenen Bedeutungen haben.

**20.** Herbizides und den Pflanzenwachstum hemmendes Mittel, dadurch gekennzeichnet, dass es als Wirkstoff mindestens ein 3-Fluor-pyridyl-2-oxy-phenoxy-Derivat der Formel I, Anspruch 1, enthält.

**21.** Die Verwendung der 3-Fluor-pyridyl-2-oxy-phenoxy-Derivate der Formel I, Anspruch 1 oder sie enthaltender Mittel zur Bekämpfung von grasartigen Unkräutern.

**22.** Die Verwendung der 3-Fluor-pyridyl-2-oxy-phenoxy-Derivate der Formel I, Anspruch 1, oder sie enthaltender Mittel, als selektive Unkrautmittel in Kulturpflanzungen.

**23.** Die Verwendung der 3-Fluor-pyridyl-2-oxy-phenoxy-Derivate der Formel I, Anspruch 1, oder sie enthaltender Mittel zur Hemmung des Pflanzen-, insbesondere des Gräserwuchses.

## Claims

1. 3-Fluoropyridyl-2-oxyphenoxy derivatives of the formula I

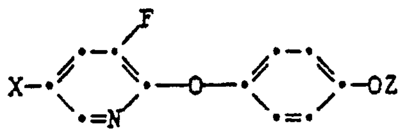

(I)

wherein

| | |
|---|---|
| X | is a halogen atom or the trifluoromethyl group, and |
| Z | is an alkanecarboxylic acid radical $-CH(CH_3)Z^1$, $CH_2Z^2$ or $CH(CH_2OCH_3)Z^2$ in which |
| $Z^1$ | is a radical $-COQ-G-Y^2$, |
| $Z^2$ | is the same as $Z^1$, |
| $Y^2$ | is a radical $-PO(OR^{14})OR^{15}$ or $SiA^7(O)_pR^{12})R^{13}$, |
| Q | is oxygen or sulfur, |
| $R^{12}$ | and $R^{13}$ are each $C_1$-$C_6$alkyl, |
| $R^{14}$, | $R^{15}$ independently of one another are each hydrogen, $C_1$-$C_4$alkyl, $C_3$-$C_4$alkenyl, $C_3$-$C_4$alkynyl, $C_2$-$C_4$halo-alkyl, $C_1$-$C_4$alkoxy-($C_2$-$C_4$)-alkyl, $C_1$-$C_4$cyanoalkyl, or a phenyl, phenyl-($C_1$-$C_4$)-alkyl or naphthyl radical which is unsubstituted or mono- or disubstituted by halogen, $C_1$-$C_4$alkyl, $C_1$-$C_4$alkoxy, $C_1$-$C_4$haloalkyl, $C_1$-$C_4$haloalkoxy, nitro or by cyano; where p is 0 or 1, and $R^{15}$ is not H when p is 0, |
| $A^7$ | is $C_1$-$C_6$alkyl, $C_1$-$C_6$alkoxy, |
| G | is a $C_1$-$C_3$alkylene chain which may be mono- or disubstituted by a substituent from the group comprising methyl and phenyl. |

2. 3-Fluoropyridyl-2-oxyphenoxy derivatives according to claim 1 of the formula (Ib)

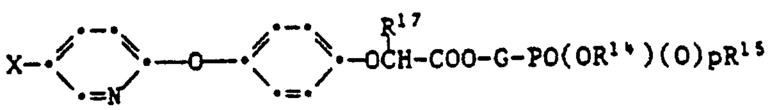

(Ib)

wherein X, G, $R^{14}$, $R^{15}$ and p have the meanings defined in claim 1, and $R^{17}$ is hydrogen, methyl or methoxymethyl.

3. 3-Fluoropyridyl-2-oxyphenoxy derivatives according to claim 2 of the formula Ib wherein X is chlorine or trifluoromethyl, G is $C_1$-$C_3$alkylene, $R_{14}$ and $R_{15}$ are each methyl or ethyl, and $R_{17}$ is methyl.

4. (2-[4(5-Chloro-3-fluoropyridyl-2-oxy)-phenoxy]-propionyloxy)-methylphosphonic acid diethyl ester according to claim 1.

5. (2-[4(5-chloro-3-fluoropyridyl-2-oxy)-phenoxy]-propionyloxy)-methylphosphonic acid dimethyl ester according to claim 1.

6. 3-Fluoropyridyl-2-oxyphenoxy derivatives according to claim 1 of the formula If

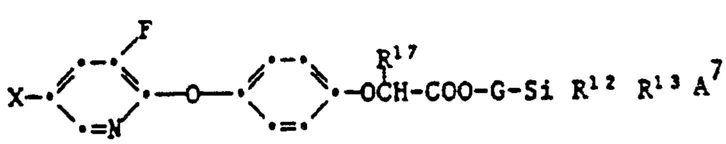

(If)

wherein $A^7$, X, G, $R^{12}$ and $R^{13}$ have the meanings defined in claim 1, and $R^{17}$ is hydrogen, methyl or methoxymethyl.

7. 3-Fluoropyridyl-2-oxyphenoxy derivatives according to claim 6 of the formula If wherein X is chlorine or

trifluoromethyl, G is $C_1$-$C_3$alkylene, and $R^{12}$, $R^{13}$ and $R^{17}$ are each methyl.

**8.** 2-[4(5-Chloro-3-fluoropyridyl-2-oxy)-phenoxy]-propionic acid trimethylsilylmethyl ester according to claim 1.

**9.** 2-[4-(5Chloro-3-fluoropyridyl-2-oxy)-phenoxy]-propionic acid 2'-trimethylsilyleth-1'-yl ester according to claim 1.

**10.** (2R)-2-[4-(5-chloro-3-fluoropyridyl-2-oxy)-phenoxy]-propionic acid trimethylsilylmethyl ester according to claim 1.

**11.** (2R)-2-[4-(5-chloro-3-fluoropyridyl-2-oxy)-phenoxy]-propionic acid 2'-trimethylsilyleth-1'-yl ester according to claim 1.

**12.** (2R)-2-[4-(3-fluoro-5-trifluoromethyl-pyridyl-2-oxy)-phenoxy]-propionic acid 2'-trimethylsilyleth-1'-yl ester according to claim 1.

**13.** (2R)-2-[4-(3-fluoro-5-trifluoromethyl-pyridyl-2-oxy)-phenoxy]-propionic acid trimethylsilylmethyl ester according to claim 1.

**14.** 2-[4(3-Fluoro-5-trifluoromethyl-pyridyl-2-oxy)-phenoxy]-propionic acid 2'-trimethylsilyleth-1'-yl ester according to claim 1.

**15.** 2-[4-(3-Fluoro-5-trifluoromethyl-pyridyl-2-oxy)-phenoxy]-propionic acid trimethylsilylmethyl ester according to claim 1.

**16.** A process for the preparation of the 3-fluoropyridyl-2-oxyphenoxy derivatives of the formula I in claim 1, which process comprises reacting a 2,3-difluoropyridine of the formula II

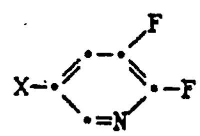

(II)

wherein X has the meaning defined in claim 1, in an inert solvent or diluent and in the presence of the equimolar amount of a base, with a 4-hydroxyphenoxy-α-alkanoic acid derivative of the formula III

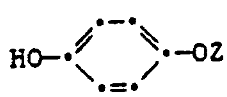

(III)

wherein Z has the meaning defined in claim 1.

**17.** A process for the preparation of the 3-fluoropyridyl-2-oxyphenoxy derivatives of the formula I, claim 1, which process comprises reacting a 4-(3-fluoropyridyl-2-oxy)-phenol of the formula IV

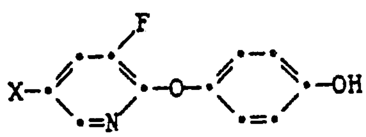

(IV)

wherein X has the meaning defined in claim 1, in an inert organic solvent or diluent and in the presence of the equimolar amount of a base, with a halide of the formula V

$$Hal - Z \qquad (V)$$

wherein Hal is chlorine or bromine, and Z has the meaning defined under the formula I, claim 1.

**18.** A process for the preparation of the 3-fluoropyridyl-2-oxyphenoxy derivatives of the formula I, claim 1, which process comprises converting a 3-aminopyridyl-2-oxyphenoxy derivative of the formula VI

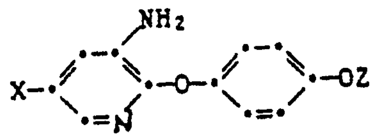

(VI)

wherein X and Z have the meanings defined under the formula I, claim 1, into a diazonium salt, and converting this further into the fluorine compound.

**19.** A process for the preparation of the 3-fluoropyridyl-2-oxyphenoxy derivatives of the formula I, claim 1, which process comprises reacting an acid halide of the formula VII

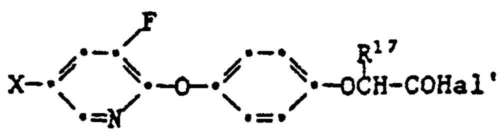

(VII)

wherein Hal' is fluorine, chlorine or bromine, X has the meaning defined in claim 1, and $R^{17}$ is hydrogen, $C_1$-$C_2$-alkyl or methoxymethyl, in an inert solvent or diluent and in the presence of an equimolar amount of a base, with an alcohol or thiol of the formula HQ-G-$Y^2$ wherein G, Q and $Y^2$ have the meanings defined under the formula I, claim 1.

**20.** A herbicidal and plant-growth-regulating composition containing, as active ingredient, at least one 3-fluoropyridyl-2-oxyphenoxy derivative of the formula I, claim 1.

**21.** The use of the 3-fluoropyridyl-2-oxyphenoxy derivatives of the formula I, claim 1, or of compositions containing them, for controlling gramineous weeds.

**22.** The use of the 3-fluoropyridyl-2-oxyphenoxy derivatives of the formula I, claim 1, or of compositions containing them, as selective herbicides in cultivated crops.

**23.** The use of the 3-fluoropyridyl-2-oxyphenoxy derivatives of the formula I, claim 1, or of compositions containing them, for inhibiting the growth of plants, especially the growth of grasses.

## Revendications

**1.** Dérivés 3-fluoro-pyridyl-2-oxy-phénoxyliques de formule I

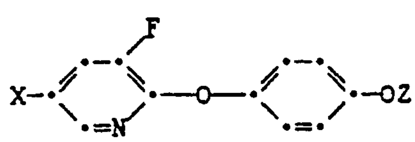

(I)

dans laquelle

X représente un atome d'halogène ou le groupe trifluorométhyle, et
Z représente un radical d'acide alcanoïque $-CH(CH_3)Z^1$, $CH_2Z_2$ ou $CH(CH_2OCH_3)Z^2$ dans lequel
Z $^1$ représente un groupe -COQ-G-$Y^2$,
$Z^2$ a la même signification que $Z^1$,
$Y^2$ représente un groupe $-PO(OR^{14})OR^{15}$ ou $SiA^7(O)pR^{12})R^{13}$

| | |
|---|---|
| Q | représente l'oxygene ou le soufre, |
| $R^{12}$ et $R^{13}$ | représentent chacun un groupe alkyle en C 1-C 6, |
| $R^{14}$, $R^{15}$ | représentent chacun, indépendamment l'un de l'autre, l'hydrogène, un groupe alkyle en C 1-C 4, alcényle en C 3-C 4, alcynyle en C 3-C 4, halogénoalkyle en C 2-C 4, (alcoxy en C 1-C 4)-alkyle en C 2-C 4, cyanalkyle en C 1-C 4 ou un groupe phényle, phényl-alkyle en C 1-C 4 ou naphtyle non substitué ou mono- à di-substitué par des halogènes, des groupes alkyle en C 1-C 4, alcoxy en C 1-C 4, halogénoalkyle en C 1-C 4, halogénoalcoxy en C 1-C 4, nitro ou cyano ; |
| p | est égal à 0 ou 1 et $R^{15}$ ne peut représenter l'hydrogène lorsque p = 0, |
| $A^7$ | représente un groupe alkyle en C 1-C 6, alcoxy en C 1-C 6, |
| G | représente une chaîne alkylène en C 1-C 3 qui peut être mono- à di-substituée par des groupes méthyle ou phényle. |

2. Dérivés 3-fluoro-pyridyl-2-oxy-phénoxyliques selon la revendication 1, répondant à la formule (Ib),

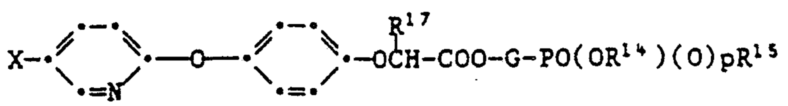

(Ib)

dans laquelle X, G, $R^{14}$, $R^{15}$ et p ont les significations indiquées dans la revendication 1 et $R^{17}$ représente l'hydrogène, un groupe méthyle ou méthoxyméthyle.

3. Dérivés 3-fluoro-pyridyl-2-oxy-phénoxyliques selon la revendication 2, répondant à la formule Ib dans laquelle X représente le chlore ou un groupe trifluorométhyle, G représente un groupe alkylène en C 1-C 3, $R^{14}$ et $R^{15}$ représentent chacun un groupe méthyle ou éthyle et $R^{17}$ un groupe méthyle.

4. Le (2-[4-(5-chloro-3-fluoro-pyridyl-2-oxy)-phénoxy]-propionyl-oxy)-méthylphosphonate de diéthyle selon la revendication 1.

5. Le (2-[4-(5-chloro-3-fluoro-pyridyl-2-oxy)-phénoxy]-propionyl-oxy)-méthylphosphonate de diméthyle selon la revendication 1.

6. Dérivés 3-fluoro-pyridyl-2-oxy-phénoxyliques selon la revendication 1, répondant à la formule If

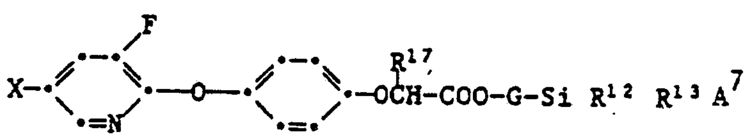

(If)

dans laquelle $A^7$ , X, G, $R^{12}$ et $R^{13}$ ont les significations indiquées dans la revendication 1 et $R^{17}$ représente l'hydrogène, un groupe méthyle ou méthoxyméthyle.

7. Dérivés 3-fluoro-pyridyl-2-oxy-phénoxyliques selon la revendication 6, répondant à la formule If dans laquelle X représente le chlore ou un groupe trifluorométhyle, G représente un groupe alkylène en C 1-C 3 et $R^{12}$ $R^{13}$ et $R^{17}$ représentent chacun un groupe méthyle.

8. Le 2-[4-(5-chloro-3-fluoro-pyridyl-2-oxy)-phénoxy]-propionate de triméthylsilyl-méthyle selon la revendication 1.

9. Le 2-[4-(5-chloro-3-fluoro-pyridyl-2-oxy)-phénoxy]-propionate de 2'-triméthylsilyl-étha-1'-yle selon la revendication 1.

10. Le (2R)-2-[4-(5-chloro-3-fluoro-pyridyl-2-oxy)-phénoxy]-propionate de triméthylsilyl-méthyle selon la revendication 1.

11. Le (2R)-2-[4-(5-chloro-3-fluoro-pyridyl-2-oxy)-phénoxy]-propionate de 2'-triméthylsilyl-étha-1'-yle selon

la revendication 1.

**12.** Le (2R)-2-[4-(3-fluoro-5-trifluorométhyl-pyridyl-2-oxy)-phénoxy]-propionate de 2'-triméthylsilyl-étha-1'-yle selon la revendication 1.

**13.** Le (2R)-2-[4-(3-fluoro-5-trifluorométhyl-pyridyl-2-oxy)-phénoxy]-propionate de triméthylsilyl-méthyle selon la revendication 1.

**14.** Le 2-[4-(3-fluoro-5-trifluorométhyl-pyridyl-2-oxy)-phénoxy]-propionate de 2'-triméthylsilyl-étha-1'-yle selon la revendication 1.

**15.** Le 2-[4-(3-fluoro-5-trifluorométhyl-pyridyl-2-oxy)-phénoxy]-propionate de triméthylsilyl-méthyle selon la revendication 1.

**16.** Procédé de préparation des dérivés 3-fluoro-pyridyl-2-oxy-phénoxyliques de formule I de la revendication 1, selon lequel on fait réagir une 2,3-difluoro-pyridine de formule II

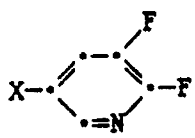

(II)

dans laquelle X a les significations indiquées dans la revendication 1, dans un solvant ou diluant inerte, en présence de la quantité équimoléculaire d'une base, avec un dérivé d'acide 4-hydroxy-phénoxy-alpha-alcanoïque de formule III

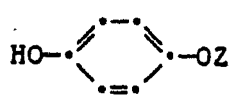

(III)

dans laquelle Z a les significations indiquées dans la revendication 1.

**17.** Procédé de préparation des dérivés 3-fluoro-pyridyl-2-oxy-phénoxyliques de formule I de la revendication 1, caractérisé en ce que l'on fait réagir un 4-(3-fluoro-pyridyl-2-oxy)-phénol de formule IV

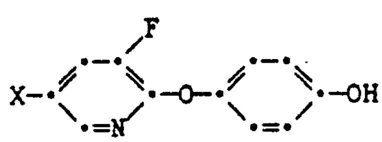

(IV)

dans laquelle X a les significations indiquées dans la revendication 1, dans un solvant ou diluant organique inerte, en présence de la quantité équimoléculaire d'une base, avec un halogénure de formule V

Hal - Z (V)

dans laquelle Hal représente le chlore ou le brome et Z a les significations indiquées en référence à la formule I dans la revendication 1.

**18.** Procédé de préparation des dérivés 3-fluoro-pyridyl-2-oxy-phénoxyliques de formule I de la revendication 1, caractérisé en ce que l'on convertit un dérivé 3-aminopyridyl-2-oxy-phénoxyliques de formule VI

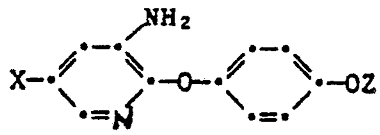

(VI)

dans laquelle X et Z ont les significations indiquées en référence à la formule I dans la revendication 1, en un sel de diazonium qu'on convertit lui-même ensuite en le dérivé fluoré.

**19.** Procédé de préparation des dérivés 3-fluoro-pyridyl-2-oxy-phénoxyliques de formule I de la revendication 1, selon lequel on fait réagir un halogénure d'acide de formule VII

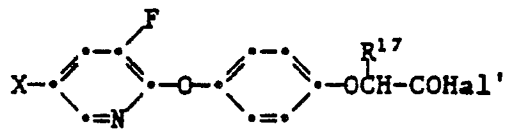

(VII)

dans laquelle Hal' représente le fluor, le chlore ou le brome, X a les significations indiquées dans la revendication 1 et $R^{17}$ représente l'hydrogène, un groupe alkyle en C 1-C 2 ou méthoxyméthyle, dans un solvant ou diluant inerte, en présence de la quantité équimoléculaire d'une base, avec un alcool ou thiol de formule HQ-G-$Y^2$ dans laquelle G, Q et $Y^2$ ont les significations indiquées en référence à la formule I dans la revendication 1.

**20.** Produit herbicide et inhibiteur de la croissance des végétaux, caractérisé en ce qu'il contient au moins une substance active consistant en un dérivé 3-fluoro-pyridyl-2-oxy-phénoxylique de formule I de la revendication 1.

**21.** L'utilisation des dérivés 3-fluoro-pyridyl-2-oxy-phénoxyliques de formule I de la revendication 1 ou de produits en contenant pour la lutte contre les graminées adventices.

**22.** L'utilisation des dérivés 3-fluoro-pyridyl-2-oxy-phénoxyliques de formule I de la revendication 1 ou de produits en contenant en tant qu'herbicides sélectifs dans des cultures de végétaux utiles.

**23.** L'utilisation des dérivés 3-fluoro-pyridyl-2-oxy-phénoxyliques de formule I de la revendication 1 ou de produits en contenant pour l'inhibition de la croissance des végétaux, en particulier des graminées.